# EUROPÄISCHE PATENTSCHRIFT

(11) **EP 2 767 244 B1**
(45) Veröffentlichungstag und Bekanntmachung des Hinweises auf die Patenterteilung: **26.04.2017**
(21) Anmeldenummer: 14154575.6
(22) Anmeldetag: 11.02.2014
(51) Int. Cl.: A61B 17/16

(54) **Chirurgisches Instrument**
Surgical instrument
Instrument chirurgical

(30) Priorität: 18.02.2013 DE 102013101594
(43) Veröffentlichungstag der Anmeldung: 20.08.2014
(73) Patentinhaber: LT technologies GmbH & Co.KG, 78573 Wurmlingen (DE)
(72) Erfinder: Arit, Matthias, 78579 Neuhausen (DE); Wiest, Claus, 78573 Wurmlingen (DE)
(74) Vertreter: Patentanwälte und Rechtsanwalt Weiß, Arat & Partner mbB

(56) Entgegenhaltungen:
- EP-B1- 1 363 542
- DE-A1-102006 012 754
- DE-A1-102008 034 287
- DE-B3- 10 320 477

## Beschreibung

Die Erfindung betrifft ein chirurgisches Instrument nach dem Oberbegriff des Anspruchs 1.

### Stand der Technik

Chirurgische Instrumente sind in vielfältiger Form und Ausführung bekannt und auf dem Markt. Die vorliegende Erfindung bezieht sich vor allem, jedoch nicht ausschliesslich, auf Schiebeschaftinstrumente, die auch als chirurgische Stanzen bezeichnet werden. Sie ist jedoch auch für andere Instrumente denkbar, bei dem ein verschiebbares Element gegenüber einem relativ feststehenden Element lageveränderbar ist, wobei in der Regel, aber auch hier wiederum nicht einschränkend, ein Maulteil betätigt wird.

In diesem Zusammenhang wird auf die DE 10 2006 012 754 A1 hingewiesen, welche ein chirurgisches Instrument offenbart. Dort ist ein Schaft und ein an dem Schaft gleitbar gelagerter Schieber vorgesehen, wobei der Schaft und der Schieber jeweils mit einem Griff verbunden sind und dabei die Griffe im Bereich des Drehgelenks lageveränderbar sind.

Weiter wird auf die EP 1 363 542 B1 verwiesen, welche ebenfalls ein Schiebeschaftinstrument offenbart, bei dem ein Anschlag derart angeordnet ist, dass er schräg in einem stumpfen Winkel von unten her gelagert ist.

Ausserdem wird auf die DE 10 2008 034 287 A1 hingewiesen, die ein chirurgisches Instrument mit den Merkmalen des Oberbegriffs des Anspruchs 1 offenbart, und auf die DE 103 20 477 B3, welche ein weiteres aus dem Stand der Technik bekanntes Schiebeschaftinstrument offenbart.

Ein derartiges chirurgisches Instrument mit einem Scherengriff ist beispielsweise aus der DE 299 22 271 U1 bekannt. Es besteht aus einer ersten Griffbranche und einer zweiten Griffbranche, wobei die erste Griffbranche relativ zur zweiten Griffbranche schwenk- bzw. spreizbar und mittels eines Federelements im Abstand voneinander haltbar ist. Mit der zweiten Griffbranche ist ein Schaft verbunden. An dem Schaft ist ein mit der ersten Griffbranche kuppelbares Schiebeelement vorgesehen, wobei das Schiebeelement in einer Stellung, in der die beiden Griffbranchen maximal voneinander beabstandet sind, von dem Schaft abnehmbar ist und in den übrigen Stellungen durch Verschwenken der ersten Griffbranche relativ zur zweiten Griffbranche auf dem Schaft axial verschiebbar ist. Etwas ähnliches ist auch aus der DE 199 49 422 A1, der DE 197 48 369 C2 oder auch der EP 1 363 542 B1 bekannt. In letzterer Schrift ist auch für eine Nutensteinschiene ein Anschlag vorgesehen, der verhindert, dass die Nutensteinschiene aus einer Führungsnut herausgleitet. Dieser Anschlag ist schräg in einem stumpfen Winkel, bevorzugt von unten her, zu der Führungsbahn geführt.

### Aufgabe

Aufgab der vorliegenden Erfindung ist es, ein chirurgisches Instrument der o.g. Art zu schaffen, welches nicht nur einfach zu zerlegen sondern auch einfach wieder zusammenzubauen ist. Ferner soll der Gedanke auch auf bisher bekannte Instrumente übertragbar sein.

### Lösung der Aufgabe

Zur Lösung der Aufgabe führen die Merkmale nach Anspruch 1.

Durch diese Anordnung wird der Zusammenbau des chirurgischen Instruments wesentlich erleichtert. Schaftteil und Schieber brauchen lediglich mit zwei Fingern zusammengedrückt zu werden, danach wird der Schieber relativ zu dem Schaftteil bewegt, bis eine Steuerkante der Ausnehmung das Rastelement überfährt und dieses in die Ausnehmung einfährt. Zum Lösung braucht lediglich das Rastelement aus der Ausnehmung gezogen zu werden, wodurch der Schieber vom Schaftteil freikommt.

Es ist auch denkbar, mehrere Rastelemente vorzusehen, die am Schaftteil angeordnet sein können.

Der Begriff Schaftteil soll ebenso wie der Begriff Schieber weit verstanden werden. Wie bereits oben erwähnt, bezieht sich die vorliegende Erfindung vor allem auf eine chirurgische Stanze, allerdings sollen vom Erfindungsgedanken auch andere chirurgische Instrumente, wie beispielsweise Rohrschaftinstrumente umfasst sein. Bei diesen ist in einem entsprechenden Rohr ein Zug- oder Druckelement verschiebbar, um ein Maul zu betätigen. Auch hier ist die erfindungsgemässe Anordnung möglich.

Das Rastelement soll automatisch beim Überfahren der Steuerkante in die Ausnehmung einrasten. Dies geschieht am einfachsten unter dem Druck eines Kraftspeichers. Dieser ist, wiederum aus Gründen der Einfachheit, eine Schraubenfeder.

Das gesamte Rastelement kann z.B. als eine Einheit ausgebildet sein und in den Schaftteil in eine entsprechend eingebrachte Bohrung eingesetzt werden. Hierbei besteht das Rastelement der Einfachheit halber aus einem Stift, der mit einem Knopf zum Betätigen verbunden ist. Der Stift stützt sich dann im Inneren eines kleinen Gehäuses gegen den Kraftspeicher ab.

Üblicherweise sind Schieber und Schaftteil bei chirurgischen Stanzen mittels einer Verbindung miteinander so gekoppelt, dass der Schieber gegenüber dem Schaftteil verschoben werden kann. Hierbei greifen Nutensteine in entsprechende hinterschnittene Nuten ein, so dass beim Verschieben kein Lösen des Schiebers vom Schaftteil stattfindet. In diesem Fall kann es sich als ratsam erweisen, das Rastelement dieser hinterschnittenen Nut zuzuordnen, d.h., das Rastelement durchsetzt die hinterschnittene Nut. Für den Nutenstein bzw. eine Nutensteinschiene ist dann, der hinterschnittenen Nut vorgeschaltet, eine längliche Ausnehmung vorgesehen, in die die Nutensteinschiene eingesetzt werden kann, so dass sie beim Verschieben des Schiebers in die hinterschnittene Nut und unter die Hinterschneidungen gleitet.

Schieber und Schaftteil sind Griffteile zugeordnet. Im vorliegenden Ausführungsbeispiel geht der Schaftteil einstückig in ein Griffteil über. Dieses Griffteil besitzt dann etwa im oberen Bereich einen Längsschlitz, durch den das Griffteil für den Schieber eingesetzt werden kann. Am Ende dieses Griffteils befindet sich eine gelenkige Verbindung mit dem Schieber, wobei die gelenkige Verbindung auch so ausgestaltet werden kann, dass der Schieber von dem Griffteil gelöst werden kann. Hierdurch wird die Reinigung des gesamten Instruments wesentlich vereinfacht.

Der vorliegende Erfindungsgedanke hat den weiteren grossen Vorteil, dass auch bereits bestehende chirurgische Instrumente, insbesondere Stanzen, durch die Anordnung des Rastelements und der mit dem Rastelement zusammenwirkenden Ausnehmung nachgerüstet werden können. Hierbei ist es ausreichend, in eine entsprechende Innenfläche des Schiebers die Ausnehmung, insbesondere ein Langloch, einzubringen und ihr gegenüberliegend in dem Schaftteil das Rastelement mit dem Stift anzuordnen. Hierbei ist nur eine Bohrung notwendig.

Ggf. ist dann noch ein Nachbearbeiten der Nutensteine bzw. Nutensteinschiene und der hinterschnittenen Nut notwendig, um sie den gewünschten Funktionen anzupassen.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
**Figur 1** eine Draufsicht auf ein erfindungsgemässes chirurgisches Instrument;
**Figur 2** eine teilweise darstellte Draufsicht auf ein Schaftteil des chirurgischen Instruments;
**Figur 3** eine teilweise dargestellte Unteransicht eines Schieberteils des chirurgischen Instruments gemäss Figur 1;
**Figur 4** eine teilweise dargestellte Seitenansicht eines Ausschnitts aus Schieber und Schaftteil;
**Figur 5** eine vergrössert dargestellte Schnittansicht eines Ausschnitts x aus dem Schaftteil gemäss Figur 4.

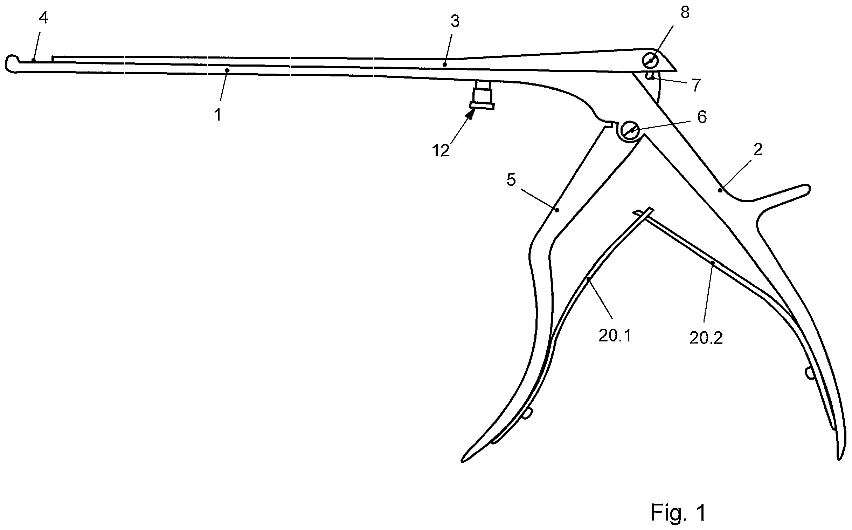

Ein erfindungsgemässes chirurgisches Instrument weist gemäss Figur 1 ein Schaftteil 1 auf, welches im gezeigten Ausführungsbeispiel einstückig in ein Griffteil 2 übergeht. Auf diesem Schaftteil 1 sitzt ein Schieber 3 auf, der entlang dem Schaftteil 1 bewegbar ist. Bei dieser Bewegung wird ein Maul 4, welches zwischen Schaftteil 1 und Schieber 3 endwärtig ausgebildet ist, geöffnet bzw. geschlossen. Hierbei kann beispielsweise ein Gewebestück ausgestanzt werden.

Die Bewegung des Schiebers 3 wird von einem weiteren Griffteil 5 bewirkt, welches das andere Griffteil 2 durchsetzt und mit diesem über ein Gelenk 6 verbunden ist. An seinem aus dem Griffteil 2 herausragenden Ende ist in dieses Griffteil 5 ein Langloch 7 eingeformt, in das ein Querbolzen 8 eingesetzt ist, so dass hier eine gelenkige Verbindung mit dem Schieber 3 gebildet wird.

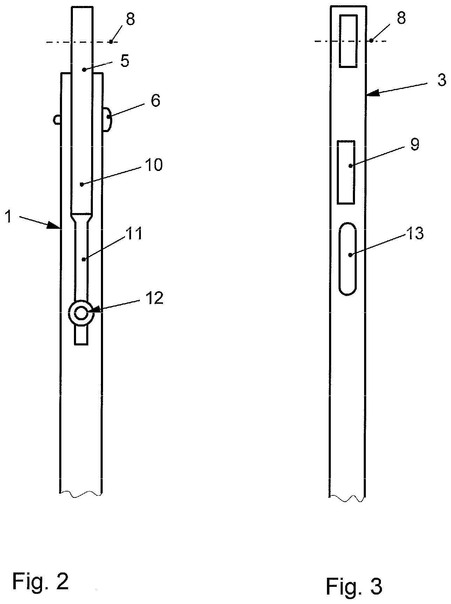

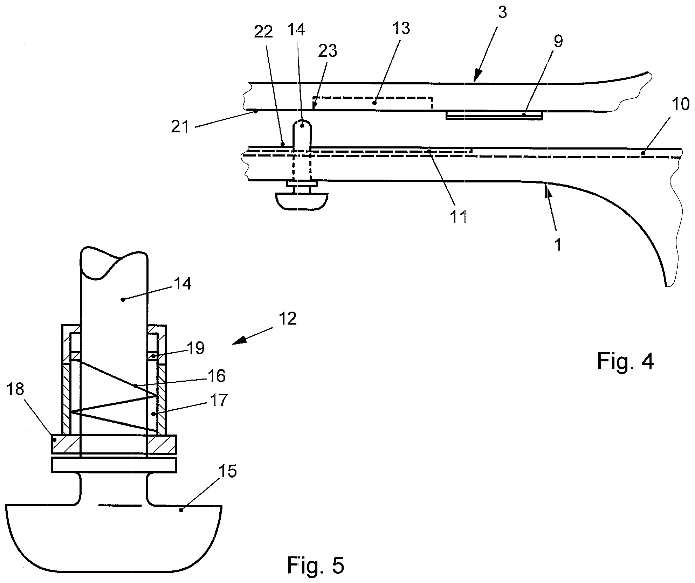

Der Schieber 3 ist mit dem Schaftteil 1 lösbar verbunden. Hierzu ragt vom Schieber 3 gemäss Figur 4 nach unten eine Nutensteinschiene 9 ab, welche in eine Ausnehmung 10 (siehe auch Figuren 2 und 3) in dem Schaftteil 1 einsetzbar ist und nach einem Schiebeweg in Richtung Maul 4 in eine hinterschnittene Nut 11 einfährt.

Um die Nutensteinschiene 9 daran zu hindern, aus der hinterschnittenen Nut 11 nach hinten vom Maul weg herauszugleiten ist eine erfindungsgemässe Rasteinrichtung 12 vorgesehen. Diese wirkt mit einem Langloch 13 im Schieber 3 zusammen. Die Rasteinrichtung 12 durchsetzt im gezeigten Ausführungsbeispiel das Schaftteil 1 im Bereich der hinterschnittenen Nut 11, sie könnte aber auch an anderer Stelle angeordnet sein. Sie weist einen Stift 14 auf, der von einem Knopf 15 betätigt werden kann. Die Betätigung geschieht gegen eine Schraubenfeder 16, die sich in einer Bohrung 17 in dem Schaftteil 1 befindet. Sie stützt sich einerseits gegen eine Wand 18 des Schaftteils 1 oder eine separat eingesetzte Platte und andererseits gegen einen Sprengring 19 ab, welcher dem Stift 14 aufgesetzt ist.

Die Funktionsweise der vorliegenden Erfindung ist folgende:
In Ausgangslage sind die beiden Griffteile 2 und 5 über das Gelenk 6, welches beispielsweise durch einen Schraubenbolzen gebildet wird, miteinander verbunden. Sie stützen sich gegenseitig durch zwei Blattfedern 20.1 und 20.2 ab, so dass das Maul 4 in Öffnungslage gehalten wird.

Schaftteil 1 und Schieber 3 befinden sich ausserhalb der Rastlage, wie dies in Figur 4 gezeigt ist. Um nun den Schieber 3 mit dem Schaftteil 1 zu verbinden, wird dieser Schieber 3 gegen das Schaftteil 1 bewegt, wobei der aus dem Schaftteil 1 herausragende Stift 14 durch eine Innenfläche 21 des Schiebers 3 weggedrückt wird. Dies geschieht gegen die Kraft der Schraubenfeder 16. Dabei fährt gleichzeitig die Nutensteinschiene 9 in die Ausnehmung 10 hinter der hinterschnittenen Nut 11 ein. In dieser Lage liegt die Innenfläche 21 plan einer Oberfläche 22 des Schaftteils 1 an.

Nunmehr wird mittels des Griffteils 5 der Schieber 3 in Richtung Maul 4 bewegt, wobei die Nutensteinschiene 9 in die hinterschnittene Nut 11 einfährt und die entsprechenden Hinterschneidungen untergreift. Damit ist der Schieber 3 gegenüber dem Schaftteil 1 festgelegt.

Nach einem weiteren Fahrweg überfährt das Langloch 13 in dem Schieber 3 den Stift 14, so dass dieser hinter einer Steuerkante 23 des Langlochs 13 in dieses einfahren kann. Hierbei entspannt sich die Schraubenfeder 16. Diese Steuerkante 23 bewirkt nun durch ein Anschlagen an dem Stift 14, dass die Spreizwirkung der Blattfeder 20.1 und 20.2 gegen die Griffteile 2 und 5 begrenzt ist. Hierdurch wird verhindert, dass die Nutensteinschiene 9 zu weit sich nach hinten bewegt und dabei unbeabsichtigt aus der hinterschnittenen Nut 11 herausfährt.

Zum Zerlegen des erfindungsgemässen chirurgischen Instruments wird einfach der Stift 14 durch Ziehen an dem Knopf 15 aus der Rastlage in dem Langloch 13 herausbewegt, so dass nunmehr die Steuerkante 23 den Stift 14 überfahren kann. Nahe einem weiteren Fahrweg des Schiebers 3 gegenüber dem Schaftteil 1 gelangt auch die Nutensteinschiene 9 ausser Eingriff mit der hinterschnittenen Nut 11. Der Schieber 3 kann nun von dem Schaftteil 1 weggeklappt werden. Ist beispielsweise das Langloch 7 nach oben hin offen, kann auch ohne Lösen des Querbolzens 8 der Schieber 3 von dem Griffteil 5 abgenommen werden.

**Bezugszeichenliste**

| | | | | | |
|---|---|---|---|---|---|
| 1 | Schaftteil | 34 | | 67 | |
| 2 | Griffteil | 35 | | 68 | |
| 3 | Schieber | 36 | | 69 | |
| 4 | Maul | 37 | | 70 | |
| 5 | Griffteil | 38 | | 71 | |
| 6 | Gelenk | 39 | | 72 | |
| 7 | Langloch | 40 | | 73 | |
| 8 | Querbolzen | 41 | | 74 | |
| 9 | Nutensteinschiene | 42 | | 75 | |
| 10 | Ausnehmung | 43 | | 76 | |
| 11 | Nut | 44 | | 77 | |
| 12 | Rasteinrichtung | 45 | | 78 | |
| 13 | Langloch | 46 | | 79 | |
| 14 | Stift | 47 | | | |
| 15 | Kopf | 48 | | | |
| 16 | Schraubenfeder | 49 | | | |
| 17 | Bohrung | 50 | | | |
| 18 | Wand | 51 | | | |
| 19 | Sprengring | 52 | | | |
| 20 | Blattfeder | 53 | | | |
| 21 | Innenfläche | 54 | | | |
| 22 | Oberfläche | 55 | | | |
| 23 | Steuerkante | 56 | | | |
| 24 | | 57 | | | |
| 25 | | 58 | | | |
| 26 | | 59 | | P | Chir. Instrument |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Chirurgisches Instrument mit einem Schaftteil (1), dem ein Schieber (3) zugeordnet ist, wobei Schaftteil (1) und Schieber (3) eine lösbare Verbindung eingehen, wobei sowohl an das Schaftteil (1) als auch an den Schieber (3) ein Griffteil (2, 5) anschliesst und die beiden Griffteile (2, 5) gelenkig miteinander verbunden sind, wobei das dem Schieber (3) zugeordnete Griffteil (5) mit diesem eine gelenkige, gegebenenfalls lösbare Verbindung eingeht, wobei von dem Schieber (3) nach unten eine Nutensteinschiene (9) abragt, welche in eine Ausnehmung (10) in dem Schaftteil (1) einsetzbar ist und nach einem Schiebeweg in Richtung eines Mauls (4) in eine hinterschnittene Nut (11) einfährt, wobei die Griffteile (2, 5) sich gegenseitig durch zwei Blattfedern (20.1, 20.2) abstützen, so dass das Maul (4) in Öffnungslage gehalten wird, wobei von dem Schaftteil (1) Rasteinrichtung (12) abragt, die beim Entlangschieben des Schiebers (3) an dem Schaftteil (1) in eine Ausnehmung (13) des Schiebers (3), bevorzugt in der Form eines Langlochs, einfährt, wobei die Rasteinrichtung (12) einen Stift (14) aufweist,
**dadurch gekennzeichnet,**
**dass** der Stift (14) von einem Knopf (15) betätigbar ist, wobei die Betätigung gegen eine Schraubenfeder (16) geschieht, die sich in einer Bohrung (17) in dem Schaftteil (1) befindet, wobei die Schraubenfeder (16). sich einerseits gegen eine Wand (18) des Schaftteils (1) und andererseits gegen einen Sprengring (19) abstützt, welcher dem Stift (14) aufgesetzt ist.

2. Instrument nach Anspruch 1, **dadurch gekennzeichnet, dass** die Rasteinrichtung (12) eine hinterschnittene Nut (11) in dem Schaftteil (1) durchsetzt.

3. Instrument nach Anspruch 1 oder 2, **dadurch gekennzeichnet, dass** an die hinterschnittene Nut (11) in dem Schaftteil (1) eine längliche Ausnehmung (10) anschliesst.

4. Instrument nach wenigstens einem der vorhergehenden Ansprüche, **dadurch gekennzeichnet, dass** die Ausnehmung (13) in dem Schieber (3) etwa gegenüberliegend der hinterschnittenen Nut (11) vorgesehen ist.

5. Instrument nach wenigstens einem der vorhergehenden Ansprüche, **dadurch gekennzeichnet, dass** an die Ausnehmung (13) in dem Schieber (3) die Nutensteinschiene (9) anschliesst.

6. Instrument nach einem der vorhergehenden Ansprüche, **dadurch gekennzeichnet, dass** die Nutensteinschiene (9) etwa gegenüber der Ausnehmung (10) in dem Schaftteil (1) vorgesehen ist.

## Claims

1. A surgical instrument with a shaft part (1) with which a slide (3) is associated, wherein the shaft part (1) and slide (3) enter into a releasable connection, wherein a grip part (2, 5) adjoins both the shaft part (1) and the slide (3) and the two grip parts (2, 5) are connected together in articulated manner, wherein the grip part (5) associated with the slide (3) enters into an articulated, optionally releasable connection therewith, wherein a groove-block rail (9) protrudes downwards from the slide (3), which rail can be inserted in a recess (10) in the shaft part (1) and after a sliding travel in the direction of a jaw (4) enters an undercut groove (11), wherein the grip parts (2, 5) are mutually supported by two leaf springs (20.1, 20.2), so that the jaw (4) is held in the opening position,
wherein
a latching means (12) protrudes from the shaft part (1), which means upon the slide (3) being pushed along the shaft part (1) enters a recess (13) in the slide (3), preferably in the form of an elongate hole, the latching means (12) having a pin (14),
**characterised in that**
the pin (14) can be actuated by a button (15), the actuation taking place against a helical spring (16) which is located in a bore (17) in the shaft part (1), the helical spring (16) being supported on one hand against a wall (18) of the shaft part (1) and on the other hand against a spring ring (19) which is placed on the pin (14).

2. An instrument according to Claim 1,
**characterised in that** the latching means (12) penetrates through an undercut groove (11) in the shaft part (1).

3. An instrument according to Claim 1 or 2, **characterised in that** an elongate recess (10) adjoins the undercut groove (11) in the shaft part (1).

4. An instrument according to at least one of the preceding claims, **characterised in that** the recess (13) is provided in the slide (3) located approximately opposite the undercut groove (11).

5. An instrument according to at least one of the preceding claims, **characterised in that** the groove-block rail (9) adjoins the recess (13) in the slide (3).

6. An instrument according to one of the preceding claims, **characterised in that** the groove-block rail (9) is provided approximately opposite the recess (10) in the shaft part (1).

## Revendications

1. Instrument chirurgical avec une partie de tige (1) à laquelle est associé à un curseur (3), la partie de tige (1) et le curseur (3) formant une connexion amovible, tant à la partie de tige (1) qu'au curseur (3) venant se raccorder une partie de préhension (2, 5) et les deux parties de préhension (2, 5) étant connectées de manière articulée l'une à l'autre, la partie de poignée (5) associée au curseur (3) formant avec ce dernier une connexion articulée éventuellement amovible, du curseur (3) faisant saillie vers le bas un rail de coulisseau (9) qui peut être placé dans un évidement (10) dans la partie de tige (1) et qui pénètre, après un trajet de coulissement en direction d'une mâchoire (4) dans une rainure en contre-dépouille (11), les parties de préhension (2, 5) se supportant mutuellement par l'intermédiaire de deux ressorts à lame (20.1, 20.2), de sorte que la mâchoire (4) soit maintenue en position ouverte,
dans lequel
de la partie de tige (1) fait saillie un dispositif d'encliquetage (12) qui pénètre, lors du passage du curseur (3) à l'endroit de la partie de tige (1), dans un évidement (13) du curseur (3), de préférence sous forme d'un trou oblong, le dispositif d'encliquetage (12) présentant une broche (14),
**caractérisé par le fait**
**que** la broche (14) peut être actionnée par un bouton (15), l'actionnement ayant lieu à l'encontre d'un ressort hélicoïdal (16) qui se trouve dans un alésage (17) dans la partie de tige (1), le ressort hélicoïdal (16) s'appuyant, d'une part, contre une paroi (18) de la partie de tige (1) et, d'autre part, contre un anneau élastique (19) qui est placé sur la broche (14).

2. Instrument selon la revendication 1, **caractérisé par le fait que** le dispositif d'encliquetage (12) traverse une rainure en contre-dépouille (11) dans la partie de tige (1).

3. Instrument selon la revendication 1 ou 2, **caractérisé par le fait que** la rainure en contre-dépouille (11) dans la partie de tige (1) vient se raccorder à un évidement allongé (10).

4. Instrument selon au moins l'une des revendications précédentes, **caractérisé par le fait que** l'évidement (13) dans le curseur (3) est prévu environ face à la rainure en contre-dépouille (11).

5. Instrument selon au moins l'une des revendications précédentes, **caractérisé par le fait qu'**à l'évidement (13) dans le curseur (3) vient se raccorder le rail de coulisseau (9).

6. Instrument selon l'une des revendications précédentes, **caractérisé par le fait que** le rail de coulisseau (9) est prévu environ face à l'évidement (10) dans la partie de tige (1).
